# EUROPÄISCHE PATENTSCHRIFT

(11) **EP 0 809 483 B1**
(45) Veröffentlichungstag und Bekanntmachung des Hinweises auf die Patenterteilung: **10.10.2001**
(21) Anmeldenummer: 96902981.8
(22) Anmeldetag: 05.02.1996
(51) Int. Cl.: A61K 7/48, A23L 3/3544, A61K 7/00

(54) **VERFAHREN ZUR HEMMUNG SORBATINDUZIERTER BRAUNVERFÄRBUNGEN IN KOSMETISCHEN MITTELN UND LEBENSMITTELN**
PROCESS FOR INHIBITING SORBATE-INDUCED BROWN DISCOLORATIONS IN COSMETICS AND FOODSTUFFS
PROCEDE D'INHIBITION DES BRUNISSEMENTS INDUITS PAR LES SORBATES DANS LES PRODUITS COSMETIQUES ET ALIMENTAIRES

(30) Priorität: 15.02.1995 DE 19504999
(43) Veröffentlichungstag der Anmeldung: 03.12.1997
(73) Patentinhaber: Nutrinova Nutrition Specialties & Food Ingredients GmbH, 65929 Frankfurt am Main (DE)
(72) Erfinder: JAGER, Martin, D-55234 Offenheim (DE)
(74) Vertreter: Schweitzer, Klaus, Dr. Dipl.-Chem.
(86) Internationale Anmeldenummer: EP9600468
(87) Internationale Veröffentlichungsnummer: WO9625140

(56) Entgegenhaltungen:
- DD-A- 158 357
- PATENT ABSTRACTS OF JAPAN vol. 018, no. 182 (C-1184), 29.März 1994 & JP,A,05 339135 (LION CORP), 21.Dezember 1993, in der Anmeldung erwähnt

## Beschreibung

Verfahren zur Hemmung sorbatinduzierter Braunverfärbungen in kosmetischen Mitteln und Lebensmitteln sowie sorbatkonservierte, farbstabilisierte Formulierungen

Die Erfindung betrifft ein Verfahren zur Herstellung sorbatkonservierter, farbstabilisierter kosmetischer Mittel und Lebensmittel sowie diese Mittel selbst.

Sorbinsäure (2,4-Hexadiensäure) und ihre Salze, im besonderen das gut wasserlösliche Kaliumsalz, werden seit vielen Jahren zur Konservierung von Lebensmitteln weltweit verwendet. Bei Sorbinsäure handelt es sich um eine ungesättigte Fettsäure, die sich durch besondere physiologische Verträglichkeit auszeichnet. Sorbinsäure wird im menschlichen Körper analog zu einer Fettsäure verstoffwechselt, akkumuliert nicht und wird von der wissenschaftlichen Beratungsremien der Weltgesundheitsorganisation und der Europäischen Union als sicher eingestuft. Der von beiden Gremien für Sorbinsäure festgelegte ADI-Wert (Acedeptable Daily Intake), der als Maß für die physiologische Unbedenklichkeit von Lebensmittelzusatzstoffen gewertet werden kann, beträgt 0 bis 25 mg/kg Körpergewicht und Tag und ist damit der mit Abstand höchste ADI-Wert aller Konservierungsstoffe. Sorbinsäure und Sorbate gelten als nicht allergen und werden daher auch in keiner der bekannten Allergiedatenbanken (z.B. Leatherhead Food Tolerance Databanks Porject) erwähnt.

Die Wirksamkeit der Sorbinsäure richtet sich vor allem gegen Hefen und Schimmelpilze und in etwas geringerem Umfang gegen Bakterien. Die Wirksamkeit der Sorbinsäure hängt vom undisoziierten Anteil und damit vom pH-Wert des zu konservierenden Gutes ab. Aufgrund des hohen pK-Wertes von 4,76 ist Sorbinsäure im Gegensatz zu anderen Konservierungsstoffen auf Basis organischer Säuren auch zur Konservierung von schwach sauren Gütern (bis pH 6,5) geeignet.

In fester Form sind Sorbinsäure und Sorbate stabil. In wäßriger Lösung, in Lebensmitteln und in kosmetischen Mitteln unterliegt Sorbinsäure jedoch oxidativen Einflüssen. Besonders durch oxidative Spaltung der Doppelbindungen können Aldehyde und Ketone entstehen, die die Ursache für off-flavours sein können. Polymerisationsprodukte dieser Aldehyde können ebenso für farbliche Veränderungen, im besonderen Bräunungsreaktionen, verantwortlich sein, wie die Reaktionsprodukte dieser Aldehyde mit Aminosäuren bzw. anderen primären und sekundären Aminogruppen. Derartige Produkte werden als Maillard-Produkte bezeichnet und sind vielfach für Farbveränderungen in kosmetischen Mitteln und Lebensmitteln verantwortlich.

Der Mechanismus der Oxidation von Sorbinsäure und entsprechende Stabilisierungsmaßnahmen sind vielfach Gegenstand wissenschaftlicher Untersuchungen gewesen (Arya, S. (1980); Stability of sorbic acid in aqueous solutions. Journal Agric. Food Chem. 28, 1246-1249; Arya. S., Thakur, B. (1988): Degradation products of sorbic acid in aqueous solutions. Food Chem. 29, 41-49; Ledward, D. (1990); Stability of sorbic acid in intermediate moisture systems. Food Add. Contam. 7, 677-683; Merciadez, M., Mohammed, K., Maniere, F. (1992): Stabilized sorbic acid or salts therefore. US Patent 966 246, 26.10.1992; Thakur, B., Singh, R., Arya, S. (1994): Chemistry of sorbates - a basic perspective. Food Rev. Intern. 10, 71-91). Im Rahmen der o.g. Untersuchungen wurde teilweise versucht, die beschriebenen sorbatinduzierten Braunverfärbungen und geruchlichen Veränderungen von Lebensmitteln durch den Zusatz von Metallionen (insbesondere Mangan) in einem Konzentrationsbereich von 0,1-5 ppm zu vermindern. Daneben ist die Farbstabilisierung von beispielsweise Süßstofflösungen auch durch Kupfer-, Zink- und Kobaltsalzen beschrieben. Der Zusatz dieser in Abhängigkeit von der Konzentration durchaus auch prooxidativ wirksamer Metalle zu Nahrungsmitteln erscheint physiologisch nicht vertretbar. Gleiches gilt für kosmetische Mittel. Daher besteht auch weiterhin die Notwendigkeit, sorbatinduzierte Braunverfärbungen sowohl in Lebensmitteln als auch in kosmetischen Mitteln zu unterbinden.

Aufgrund ihrer guten Wirksamkeit gegen kosmetikverderbende Mikroorganismen und ihrer besonderen Hautverträglichkeit und physiologischen Sicherheit wird Sorbinsäure in jüngerer Zeit verstärkt zur Konservierung kosmetischer Mittel eingesetzt. Sorbate sind die Konservierungsstoffe der Wahl für kosmetische Mittel, da:
- sie in besonderem Maße gegen kosmetikverderbende Mikroorganismen wirken (z.B. Pseudomonas aeruginosa)
- sie die Bildung von Mycotoxinen in kosmetischen Mitteln unterbinden
- sie gut haut- und schleimhautverträglich sind
- sie nicht hautreizend sind
- sie nicht phototoxisch sind
- sie keine ökologischen Probleme verursachen (Wassergefährungsklasse 0)

Basierend auf diesen ausgezeichneten physiologischen Daten wurde Sorbinsäure vom CIR-Expert Panel als "safe" eingestuft und ist weltweit für die Verwendung in kosmetischen Mitteln zugelassen.

Die technologischen Vorteile einer Sorbatverwendung in Kosmetischen Mitteln sind die folgenden:
- Schutz vor mikrobieller Kontamination während Lagerung und Gebrauch
- Gute Kompatibilität mit Kosmetikrohstoffen
- Keine Inaktivierung durch Kosmetikinhaltsstoffe
- "Synergistische" Effekte mit anderen Kosmetikkonservierungsstoffen
- Hohe Wirksamkeit bei kosmetikrelevanten pH-Werten
- Aufgrund des günstigen Verteilungskoeffizienten (Verbleiben der mikrobiologisch sensitiven Wasserphase) zur Konservierung von Öl-in-Wasser-Emulsionen besonders geeignet
- Kein Wirkungsverlust durch Reaktion mit Verpackungsmaterialien

Aufgrund dieser ausgezeichneten technologischen Eigenschaften werden Sorbate in sämtlichen kosmetischen Mitteln (z.B. Shampoos, Duschgel, Bodylotion, Sonnenschutzpräparate, Reinigungsmilch, Gesichtswasser, Selbstbräuner, dekorative Kosmetik, Mundhygieneprodukte und Feuchttücher bzw. feuchtem Toilettenpapier) eingesetzt. Es ist jedoch literaturbekannt, daß gerade in kosmetischen Mitteln, die über längere Zeit gelagert werden, einem oxidativen Einfluß ausgesetzt sind und Aminokomponenten enthalten, sorbatinduzierte Braunverfärbungen immer wieder zu Problemen führen(Domsch, A. (1994): Die kosmetischen Präparate, Band 2: Wäßrige und tensidhaltige Formulierungen, 4. Auflage, S. 329, Augsburg: Verlag für die chemische Industrie). Die bisher am häufigsten durchgeführte protektive Maßnahme ist die Mitverwendung von komplexierenden Agenzien (EDTA oder Citraten), die durch Komplexierung prooxidativer Metallionen sorbatinduziert Braunverfärbungen verlangsamen. Eine zweite Möglichkeit stellen Modifikationen der Parfümierung dar. Durch beide Maßnahmen ist eine Verzögerung sorbatinduzierter Braunverfärbungen erreichbar. Besonders für klare Formulierungen in klaren Kunststoff-Flaschen (eine Formulierung die z. Zt. sehr häufig verwendet wird) bleibt das genannte Problem jedoch bestehen. Da beschriebene Braunverfärbungen temperaturabhängig sind, gilt dies im besonderen für in tropische Länder exportierte kosmetische Mittel.

Da Sorbate aufgrund ihrer antimikrobiellen Aktivität auch als Wirkstoffe in Mundspülungen, Zahnpasten und Reinigungsmitteln für dritte Zähne verwendet werden können und diese Produkte häufig als klare Formulierungen angeboten werden, ist die Verwendung von Allantoin als Wirkstoff (entzündungshemmend, schmerzlindernd) und Inhibitor sorbatverursachter Braunverfärbungen in Mundhygienemitteln beschrieben (Hirohata, H., Ozawa, T. (1993): Composition for dental use. JP 5-339135). Mundhygienemittel stellen jedoch, da sie im allgemeinen keine die Verfärbung beschleunigenden Aminokomponenten enthalten, keinen allzu hohen Anspruch an die bräunungsinhibierende Substanz.

Da oxidativ gebildete Abbauprodukte der Sorbinsäure in besonderem Maße mit ernährungsphysiologisch hochwertigen Lebensmittelbestandteilen wie Aminosäuren reagieren können, ist der Schutz von Sorbinsäure vor oxidativen Veränderungen auch von erheblicher ernährungsphysiologischer Relevanz. Untersuchungen die eine Reaktivität der Sorbinsäure-Abbauprodukte mit Aminosäuren wie Lysin oder Glutaminsäure beschreiben, liegen vor (Ledward, A.D: (1990): Stability of sorbic acid in intermediate moisture systems, Food Add. Contam. 7, 677-683).
Es bestand also Bedarf an einem Verfahren, das es ermöglicht, kosmetische Mittel und Lebensmittel mit Sorbinsäure zu konservieren, ohne daß Braunfärbungen oder sensorisch relevante Veränderungen auftreten.

Diese Aufgabe wird gelöst durch ein Verfahren zur visuellen und sensorischen Stabilisierung sorbatkonservierter, Aminosäuren enthaltender Lebensmittel und kosmetischer Mittel, dadurch gekennzeichnet, daß man diesen Produkten Allantoin und Citrate als Bräunungsinhibitoren zusetzt.

Die sorbatkonservierten Lebensmittel und kosmetischen Mittel können hierbei den Konservierungsstoff sowohl in Form von freier Sorbinsäure als auch als deren physiologisch unbedenkliche Salze wie z.B. Kalium- oder Cacliumsorbat enthalten. Die verwendeten Sorbatkonzentrationen, berechnet als Sorbinsäure, liegen für Lebensmittel und kosmetische Mittel im allgemeinen zwischen 0.005 % und 5 %.

Durch Zusatz von Allantoin, einem aufgrund seiner entzündungshemmenden, keratinolytischen und wundheilenden Eigenschaften in der Kosmetik häufig verwendeten Wirkstoff lassen sich überraschenderweise sorbatinduzierte Verfärbungen und geruchliche Veränderungen kosmetischer Mittel jeglicher Art drastisch minimieren. Werden zusätzlich Citrate (z.B. Citronensäure oder Di-Natriumcitrat) als komplexierende Agentien eingesetzt, lassen sich Sorbatinduzierte Verfärbungen nahezu gänzlich unterbinden. Die üblichen Einsatzkonzentrationen an Sorbaten in kosmetischen Mitteln, berechnet als Sorbinsäure, sind in der folgenden Tabelle zusammengefaßt. Bei Mitverwendung anderer Kosmetikkonservierungsstoffe liegen die Konzentrationen entsprechend niedriger.

**Tabelle 1**

| Übliche Anwendungskonzentrationen von Sorbinsäure in kosmetischen Mitteln | | | |
|---|---|---|---|
| Produktklasse | Art der Konservierung | pH-Wert | Sorbinsäurekonzentration (in Gew.-%) |
| Shampoo | Sorbinsäure Sorbinsäure u.a. | 4,8 - 5,5 4,8 - 5,6 | 0,15 - 0,3 < 0,1 - 0,2 |
| Duschgel | Sorbinsäure Sorbinsäure u.a. | 4,8 - 5,5 4,8 - 5,5 | < 0,1 - 0,2 0,15 - 0,35 |
| Body Lotion | Sorbinsäure Sorbinsäure u.a. | 5,0 - 6,0 5,0 - 6,0 | 0,1 - 0,2 < 0,1 |
| Sonnenschutzmilch | Sorbinsäure u.a. | 5,2 - 5,6 | 0,1 - 0,2 |
| Reinigungsmilch | Sorbinsäure u.a. | 5,8 - 6,2 | < 0,1 - 0,2 |
| Gesichtswasser | Sorbibsäure u.a. | 5,80 | < 0,1 |
| Selbstbräuner | Sorbinsäure u.a. | 4,90 | < 0,1 |
| dekorative Kosmetik | Sorbinsäure u.a. | 6,2 - 7,0 | 0,1 - 0,2 |
| Mundhygiene | Sorbinsäure | 6,5 - 6,6 | 0,20 |
| Feuchttücher | Sorbinsäure | 5,5 - 5,9 | 0,1 - 0,15 |
| | Sorbinsäure u.a. | 5.50 | 0,10 |

Allantoin wird vorteilhaft in einer Größenordnung von 0,01 bis 10 Gew.-%, insbesondere 0,05 bis 5 Gew.-%, bevorzugt 0,1 bis 3 Gew.-%, besonders bevorzugt 0,2 bis 2 Gew.-% eingesetzt. Für die Citrate empfiehlt sich eine Größenordnung von 0,05 bis 5 Gew.-%, insbesondere 0,1 bis 3 Gew.-%, bevorzugt 0,2 bis 2 Gew.-% und besonders bevorzugt 0,2 bis 1 Gew.-%. Gute Ergebnisse werden erzielt, wenn die Allantoinkonzentrationen im Vergleich zu den verwendeten Sorbatkonzentrationen 30 bis 200 Gew.-%, insbesondere 40 bis 150 Gew.-% und bevorzugt 50 bis 100 Gew.-% betragen. Für besonders bräunungsanfällige Formulierungen können entsprechend höhere Allantoinkonzentrationen gewählt werden. Citrate sollten auf prozentualer Basis in der gleichen Konzentration wie die Sorbate verwendet werden. Dienen Citrate dem Zweck der pH-Regulierung, können auch höhere Konzentrationen eingesetzt werden.

Besonders überraschend erscheint die Tatsache, daß Allantoin, obwohl es drei sekundäre und eine primäre Aminogruppe enthält, in der Lage ist, sorbatinduzierte Verfärbungen in kosmetischen Mitteln und Lebensmitteln zu unterbinden. Gerade Aminogruppen gelten als zusätzlicher "Risikofaktor" bezüglich sorbatinduzierter Verfärbungen. Darüber hinaus liegen keine Befunde aus der Literatur vor, daß Allantoin über ein nennenswertes antioxidatives Potential (z.B. Quenching von Singulett-Sauerstoff oder Sauerstoff- bzw. Hydroxyl-Radikalen) verfügt. Vielmehr scheint es so zu sein, daß die Reaktionsprodukte zwischen den Sorbatabbauprodukten und Allantoin überraschenderweise nicht gefärbt sind und keine sensorischen Probleme hervorrufen. Bedenkt man die Eigenfärbung von Reaktionsprodukten zwischen Sorbat-Abbauprodukten und Aminosäuren ist diese Eigenschaft (Farblosigkeit) von Sorbat-Allantoin-Addukten besonders bemerkenswert.

Die Schutzwirkung von Allantoin, Citraten sowie den entsprechenden Synergismen in wäßrigen Lösungen zeigen die Tabellen 2 und 3.

**Tabelle 2:**

| Sorbatbedingte Verfärbungen wäßriger Lösungen (40°C, bis 6 Monate) | | | | |
|---|---|---|---|---|
| Kaliumsorbat (%) | Allantoin (%) | Dinatriumcitrat (%) | Lagerzeit Monate | Extinktion |
| 0,10 | - | - | 1 | 0,12 |
| | | | 2 | 0,10 |
| | | | 3 | 0,53 |
| | | | 6 | 1,04 |
| 0,10 | 0,05 | - | 1 | 0,09 |
| | | | 2 | 0,10 |
| | | | 3 | 0,19 |
| | | | 6 | 0,26 |
| 0,10 | 0,10 | - | 1 | 0,06 |
| | | | 2 | 0,09 |
| | | | 3 | 0,11 |
| | | | 6 | 0,18 |
| 0,10 | - | 0,05 | 1 | 0,10 |
| | | | 2 | 0,16 |
| | | | 3 | 0,36 |
| | | | 6 | 0,72 |
| 0,10 | | 0,10 | 1 | 0,08 |
| | | | 2 | 0,014 |
| | | | 3 | 0,21 |
| | | | 6 | 0,46 |
| 0,10 | 0,10 | 0,10 | 1 | 0,06 |
| | | | 2 | 0,07 |
| | | | 3 | 0,07 |
| | | | 6 | 0,09 |

**Tabelle 3:**

| Sorbatbedingte Verfärbungen wäßriger Lösungen (40°C, bis 6 Monate) | | | | |
|---|---|---|---|---|
| Kaliumsorbat (%) | Allantoin (%) | Dinatriumcitrat (%) | Lagerzeit Monate | Extinktion |
| 0,50 | - | - | 1 | 0,20 |
| | | | 2 | 0,35 |
| | | | 3 | 0,81 |
| | | | 6 | 1,32 |
| 0,50 | 0,50 | - | 1 | 0,12 |
| | | | 2 | 0,21 |
| | | | 3 | 0,29 |
| | | | 6 | 0,41 |
| 0,50 | - | 0,50 | 1 | 0,18 |
| | | | 2 | 0,29 |
| | | | 3 | 0,43 |
| | | | 6 | 0,86 |
| 0,50 | 0,50 | 0,50 | 1 | 0,06 |
| | | | 2 | 0,10 |
| | | | 3 | 0,16 |
| | | | 6 | 0,22 |

Unter Zusatz einer Aminosäure, z.B. Alanin, wird die Verfärbung der Kontrolle (ohne Allantoin und Citrate) erwartungsgemäß beschleunigt, während sich die Schutzwirkung von Allantoin bzw. der synergistischen Mischung Allantoin und Citrat erneut dokumentiert (s. Tabelle 4).

**Tabelle 4:**

| Sorbatbedingte Verfärbungen wäßriger aminosäurehaltiger Lösungen (40°C, bis 6 Monate) | | | | | |
|---|---|---|---|---|---|
| Kaliumsorbat (%) | Allantoin (%) | Dinatriumcitrat (%) | Alanin (%) | Lagerzeit Monate | Extinktion |
| 0,50 | - | - | 0,50 | 1 | 0,33 |
| | | | | 2 | 0,57 |
| | | | | 3 | 0,96 |
| | | | | 6 | 1,56 |
| 0,50 | 0,50 | 0,50 | 0,50 | 1 | 0,11 |
| | | | | 2 | 0,15 |
| | | | | 3 | 0,19 |
| | | | | 6 | 0,33 |

### Beispiele

Die folgenden Beispiele zeigen sorbatkonservierte kosmetische Mittel, die unter Verwendung von Allantoin bzw. Allantoin und Citraten hergestellt und gelagert wurden. Entsprechende Kontrollen (ohne Allantoin bzw. Allantoin und Citrat) weisen nach gleicher Lagerzeit und Lagerbedingungen (6 Monate, 40°C) bedeutend intensivere Verfärbungen auf.

### (In Klammern sind jeweils die INCI-Bezeichnungen der Substanzen angegeben)

### Beispiel 1

| Haarshampoo klar, 16,9 % WAS | | |
|---|---|---|
| A | Na-Salz-Alkyldiglykolethersulfat | |
| | (Sodium Laureth Sulfate) | % |
| | flüssig | 40,00 |
| | Parfümöl | 0,3 |
| B | Wasser | 52,50 |
| | Natriumhydroxid | 0,2 |
| C | Alkylethercarbonsäure | 4,0 |
| | (Laureth Carboxylic Acid) | |
| | Fettalkoholpolyglykolether | 2,0 |
| | (Laureth-3) | |
| | Farbstofflösung | q.s. |
| | Kaliumsorbat | 0,25 |
| | Dinatriumcitrat | 0,4 |
| | Allantoin | 0,2 |
| D | Natriumchlorid | 1,0 |

### Herstellung:

I: Die Komponenten von A zusammen. II: B lösen, in I einrühren. III: Die Komponenten von C nacheinander in I einrühren. IV: Gegebenenfalls den pH-Wert regulieren. V: Abschließend die Viskosität mit D einstellen.

### Beispiel 2

| Cremespülung | | |
|---|---|---|
| | | % |
| A | Dialkyldimethylammoniumchlorid | 1,5 |
| | (Distearyldiammonium Chloride) | |
| | Fettalkoholpolyglykolether | 1,5 |
| | (Ceteareth 3) | |
| | Cetylalkohol | 2,5 |
| | Paraffinöl, hochviskos | 1,0 |
| B | Alkylpolyethoxyammoniumlactat | 2,0 |
| | (PEG-5-Stearyl-Ammoniumlactat) | |
| | Wasser | 91,00 |
| | Kaliumsorbat | 0,3 |
| C | Parfümöl | 0,3 |
| | Farbstofflösung | q.s. |
| | Allantoin | 0,3 |
| D | Citronensäure --> pH 4,0 | q.s. |

Herstellung: I: A bei ca. 75°C aufschmelzen. II: B auf ca. 75°C aufwärmen. III: II unter Rühren I zusetzen und kaltrühren. IV: Bei ca 40°C die Komponenten von C in III einrühren. V: Den pH-Wert mit D einstellen.

### Beispiel 3

| Waschlotion klar, 10.5 % WAS | | |
|---|---|---|
| | | % |
| A | Acylamidopolyglykolethersulfat-Mg-Salz | 20,00 |
| | (Magnesium-PEG-3-Cocamide Sulfat) | |
| | Acylglutamat-Mono-Na-Salz | 6,0 |
| | (Sodium Cocoyl Glutamate) | |
| | Glucamate DOE 120 | 1,0 |
| | Parfümöl | 0,3 |
| | Wasser | 62,40 |
| | Kaliumsorbat | 0,3 |
| | Dinatriumcitrat | 0,3 |
| | Allantoin | 0,2 |
| B | Fettsäureamidoalkylbetain | 10,00 |
| | (Cocamidopropylbetain) | |

Herstellung: I: Die Komponenten von A zusammenrühren. II: Gegebenenfalls den pH-Wert regulieren, dann die Viskosität mit B einstellen.

### Beispiel 4

| Duschbad 2 in 1, 18,3 % WAS | | |
|---|---|---|
| | | % |
| A | Wasser | 45,90 |
| | Allantoin | 0,4 |
| | Polymer IR 400 | 0,2 |
| | Fettsäureisothionat-Na-Salz | 4,0 |
| | (Sodium Cocoyl Isothionate) | |
| B | Alkyldiglykolethersulfat-Na-Salz | |
| | (Sodium Laureth-Sulfate) | |
| | flüssig | 30,00 |
| | Acylglutamat-Mono-Na-Salz | 5,0 |
| | (Sodium Cocoyl Glutamat) | |
| | Cetiol HE | 2,0 |
| C | Alkylethersulfat mit Perlglanzmittel | 4,0 |
| | (Sodium Laureth Sulfate and PEG-3 Distearate) | |
| | Parfümöl | 0,5 |
| | Fettalkoholpolyglykolether | 2,0 |
| | (Laureth-3) | |
| | Fettsaäureamidoalkylbetain | 5,0 |
| | (Cocamidopropylbetain) | |
| | Kaliumsorbat | 0,25 |
| | Dinatriumcitrat | 0,3 |
| D | Natriumchlorid | 0,7 |

Herstellung: I: Die Komponenten von A unter Rühren und ca. 60°C Erwärmen lösen. II: B in I einrühren. Schaumbildung ist zu vermeiden. III: Abkühlen lassen und bei ca. 35°C einrühren. IV: Gegebenenfalls den pH-Wert regulieren, anschließend die Viskosität mit D einstellen.

### Beispiel 6

| Sonnenschutzcreme, Typ O/W | | |
|---|---|---|
| | | % |
| A | Glycerinmonodistearat (Glyceryl Stearat SE) | 12,00 |
| | Cetyl/Stearylalkohol mit 6 EO | 1,0 |
| | (Ceteareth 6 and Stearyl Alcohol) | |
| | Cetyl/Stearylalkohol mit 25 EO | 1,0 |
| | (Ceteareth 25 and Stearyl Alcohol) | |
| | Capryl/Caprinsäuretriglycerid | 10,00 |
| | (Caprylic/Capric Triglyceride) | |
| | PPG-Myristylether (PPG-3-Myristyl Ether) | 10,00 |
| | 2,4,6-Trianilo-p-(certo-2'-ethylhexyl-1'-oxi)-1,3,5-triazin (Octyl Triazone) | 3,0 |
| B | p-Aminobenzoesäureethylester (PEG-25 PABA) | 5,0 |
| | Glycerin 87 % | 3,0 |
| | Kaliumsorbat | 0,4 |
| | Allantoin | 0,4 |
| | Wasser dest. | 55.00 |
| C | Parfümöl | q.s. |

Herstellung: Phase A und B getrennt auf ca. 80°C erwärmen. Phase B in Phase A unter Homogenisieren einrühren, kurz nachhomogenisieren. Abkühlen auf ca. 40°C, Phase C zugeben, nochmals homogenisieren.

### Beispiel 7

| Zahnspülung gegen Zahnbelag | | |
|---|---|---|
| | | % |
| A | Natrosol 250 | 0,3 |
| | Wasser dest. | 40,00 |
| B | PEG-40 hydriertes Rizinusöl mit 10 % H₂O | 2,5 |
| | (PEG-40 Hydrogenated Castor Oil) | |
| | (-)-Alpha-Bisabolol | 0,02 |
| | Aromaöl | 0,4 |
| | Ethanol 96 % | 7,5 |
| | Glycerin 87 % | 8,0 |
| | Allantoin | 0,4 |
| | Natriumlaurylsulfat (C₁₂-C₁₆) | 0,4 |
| | (Sodium Lauryl Sulfate) | |
| | Acesulfam K | 0,15 |
| | Natriumhydrogencarbonat | 1,6 |
| | Kaliumsorbat | 0,9 |
| | Natriumsalicylat | 0,4 |
| | Natriumtetraborat | 0,4 |
| | Wasser dest. | q.s. |

Herstellung: Phase A quellen lassen. Phase B klar lösen. Phase A in Phase B einrühren.

## Patentansprüche

1. Verfahren zur visuellen und sensorischen Stabilisierung sorbatkonservierter, Aminosäuren enthaltender Lebensmittel und kosmetischer Mittel, **dadurch gekennzeichnet, daß** man diesen Produkten Allantoin und Citrate als Bräunungsinhibitoren zusetzt.

2. Verfahren nach Anspruch 1, **dadurch gekennzeichnet, daß** die sorbatkonservierten Lebensmittel und kosmetischen Mittel den Konservierungsstoff sowohl in Form von Sorbinsäure als auch als deren physiologisch unbedenkliche Salze, insbesondere Kalium- oder Calciumsorbat enthalten.

3. Verfahren nach Anspruch 1 oder 2, **dadurch gekennzeichnet, daß** die verwendeten Sorbatkonzentrationen, berechnet als Sorbinsäure, für Lebensmittel und kosmetische Mittel zwischen 0,005 und 5 Gew.-% betragen.

4. Verfahren nach den Ansprüchen 1 bis 3, **dadurch gekennzeichnet, daß** Allantoinkonzentrationen zwischen 0,001 und 10 Gew.-% insbesondere zwischen 0,05 und 5 Gew.-%, bevorzugt zwischen 0,1 und 3 Gew.-%, besonders bevorzugt zwischen 0,2 und 2 Gew.-% sowie Citratkonzentrationen zwischen 0,05 und 5 Gew.-%, insbesondere zwischen 0,1 bis 3 Gew.-%, bevorzugt zwischen 0,2 und 2 Gew.-%, besonders bevorzugt zwischen 0,2 bis 1 Gew.-% verwendet werden.

## Claims

1. A method for visual and sensorial stabilization of foodstuffs and cosmetic compositions containing sorbate preservative and amino acids, which comprises adding allantoin and citrates to these products as browning inhibitors.

2. The method according to claim 1, wherein the foodstuffs and cosmetic compositions containing sorbate preservative comprise the preservative both in the form of sorbic acid and as a physiologically acceptable salt thereof, in particular potassium sorbate or calcium sorbate.

3. The method as claimed in claim 1 or 2, wherein the sorbate concentrations used, calculated as sorbic acid, for foodstuffs and cosmetic compositions are between 0.005 and 5% by weight.

4. The method as claimed in any of claims 1 to 3, wherein allantoin concentrations of between 0.001 and 10% by weight, in particular between 0.05 and 5% by weight, preferably between 0.1 and 3% by weight, particularly preferably between 0.2 and 2% by weight, and citrate concentrations of between 0.05 and 5% by weight, in particular between 0.1 and 3% by weight, preferably between 0.2 and 2% by weight, particularly preferably between 0.2 and 1% by weight, are used.

## Revendications

1. Procédé pour la stabilisation visuelle et sensorielle de produits alimentaires et de cosmétiques contenant des aminoacides, conservés à l'aide de sorbates, **caractérisé en ce qu'**on ajoute à ces produits de l'allantoïne et des citrates en tant qu'inhibiteurs de brunissement.

2. Procédé selon la revendication 1, **caractérisé en ce que** les produits alimentaires et les cosmétiques conservés à l'aide de sorbates contiennent le conservateur sous forme d'acide sorbique ainsi que de ses sels physiologiquement acceptables, en particulier le sorbate de calcium ou de potassium.

3. Procédé selon la revendication 1 ou 2, **caractérisé en ce que** les concentrations de sorbate utilisées, calculées en tant qu'acide sorbique, sont comprises entre 0,005 et 5 % en poids pour des produits alimentaires ou des cosmétiques.

4. Procédé selon les revendications 1 à 3, **caractérisé en ce qu'**on utilise des concentrations d'allantoïne comprises entre 0,001 et 10 % en poids, en particulier entre 0,05 et 5 % en poids, de préférence entre 0,1 et 3 % en poids, de façon particulièrement préférée, entre 0,2 et 2 % en poids, ainsi que des concentrations de citrate comprises entre 0,05 et 5 % en poids, en particulier entre 0,1 et 3 % en poids, de préférence entre 0,2 et 2 % en poids, de façon particulièrement préférée, entre 0,2 et 1 % en poids.
